# EUROPEAN PATENT SPECIFICATION

(11) **EP 2 752 178 B1**
(45) Date of publication and mention of the grant of the patent: **04.10.2017**
(21) Application number: 12828930.3
(22) Date of filing: 23.08.2012
(51) Int. Cl.: A61F 13/15, A61F 13/49

(54) **ABSORBENT ARTICLE MANUFACTURING DEVICE**
VERFAHREN ZUR HERSTELLUNG EINES SAUGFÄHIGEN ARTIKELS
DISPOSITIF DE FABRICATION D'ARTICLE ABSORBANT

(30) Priority: 31.08.2011 JP 2011189114
(43) Date of publication of application: 09.07.2014
(73) Proprietor: Unicharm Corporation, Ehime 799-0111 (JP)
(72) Inventor: ISHIKAWA, Shinichi, Kanonji-shi Kagawa 769-1602 (JP)
(74) Representative: Dolleymores
(86) International application number: PCT/JP2012/071339
(87) International publication number: WO 2013/031648

(56) References cited:
- EP-A1- 1 428 487
- WO-A1-2010/113856
- JP-A- S 648 184
- JP-A- S6 438 377
- JP-A- S6 452 801
- JP-A- S61 162 462
- JP-A- 2004 329 873
- JP-B2- 3 466 230
- US-A- 4 578 133
- US-A1- 2003 010 423
- US-A1- 2011 188 985

## Description

### [Technical Field]

The present invention relates to a device for manufacturing an absorbent article, configured to arrange elastic members on a web in which components configuring a part of the absorbent articles are conveyed in continuity.

### Background Art

Conventionally, in a step of manufacturing absorbent articles, in order to improve the fitting feeling of leg-hole regions to a wearer, such a method has been widely used that elastic members are arranged in positions corresponding to the leg-hole regions.

Specifically, in this manufacturing step, the absorbent articles provided with leg gathers in accordance with shapes of the leg-hole regions are continuously manufactured by arranging the elastic members configuring the leg gathers in a curved shape in positions corresponding to the leg-hole regions on the web in which components configuring the absorbent articles are in continuity.

Patent Document 1 and Patent Document 2, for example, disclose a technique of continuously arranging the elastic members configuring the leg gathers on the web. Patent Document 1 discloses a manufacturing device provided with a rotation roller which rotates around a rotation axis to convey a band-shaped elastic member along an outer circumference surface. The rotation roller has a cross-sectional shape, which changes along an axial direction of the rotation axis, and serves as a crown roller having the largest diameter at the central portion in the axial direction. The rotation roller rolls on a planar base body while swinging in a crossing direction crossing a conveyance direction.

The elastic member is conveyed along the outer circumference surface in accordance with rotation of the rotation roller and is shifted in the crossing direction in accordance with swing of the rotation roller. Therefore, according to the manufacturing device in Patent Document 1, the band-shaped elastic member is conveyed while being displaced in the crossing direction, thereby being able to continuously arrange the elastic members in a curved shape.

Further, Patent Document 2 discloses a method of arranging the elastic members intermittently so as not to extend over the entire absorbent article in a longitudinal direction but to extend correspondingly to only the leg-hole regions. Specifically, the elastic member is continuously pasted on the web in a slacked state in the conveyance direction and thereafter, the slacked web is stretched so that the elastic member is divided and arranged intermittently.
Patent Document 3 discloses a method for producing a wearing article and includes the steps of: supplying an elastic member; stretching the elastic member; placing the stretched elastic member so that the elastic member spreads across a plurality of first webs divided in a transport direction; making a part of a second web loose in the transport direction while transporting the second web, thereby forming a loose portion; placing the first webs, on which the elastic member is disposed, on non-loose portions before and after the loose portion of the second web; and cutting the elastic member between adjacent first webs of the plurality of first webs.
Patent Document 4 relates to a manufacturing method of a composite sheet of an absorbent article, the method includes joining a continuous body of an elastic member in a predetermined meander pattern to a continuous body of a sheet transported continuously in a transporting direction by feeding the continuous body of the elastic member to the continuous body of the sheet via an oscillating arm that oscillates in an intersecting direction intersecting the transporting direction with a spindle portion as a swivel fulcrum.

### [Related Art Document]

### [Patent Document]

Patent Document 1: Japanese Unexamined Patent Application Publication (Translation of PCT Application) No. 2003-517880 (Figs. 1 and 4)
Patent Document 2: Japanese Patent No. 3466230 (Figs. 2, 6, and 7)
Patent Document 3: US 2011/188985 A1
Patent Document 4: WO 2010/113856 A1 (EP 2415434 A1)

### [Summary of Invention]

### [Problem to Be Solved by the Invention]

According to the method described above, the elastic members can be arranged around the legs by being continuously installed in a curved shape. However, further improvement has recently been requested in the feeling of comfort at the time of wearing the absorbent article, and it is desired that the elastic members be arranged in a more complicated curved shape, only in parts corresponding to necessary portions, that is, the leg-hole regions.

For example, the manufacturing device in Patent Document 1 may not achieve a fine curved shape due to the shifting of the elastic members in the crossing direction based on the displacement of the outer circumference surface in association with the swing of a rotation body. Further, with the manufacturing device in Patent Document 1, the elastic members cannot be arranged intermittently in the leg-hole regions only, so that the fitting feeling cannot be improved enough to achieve further improvement in the feeling of comfort at the time of wearing the absorbent article.

On the other hand, according to the method described in Patent Document 2, the elastic members can be arranged in the leg-hole regions only. However, the elastic member is cut by stretching the web, so that the ends of the elastic member get shrunken, and can neither be positioned nor installed to a proper position. Therefore, it is impossible to achieve a fine curved shape and a further improvement in the feeling of comfort at the time of wearing the absorbent article.

The present invention has been achieved in consideration of such circumstances, and an object thereof is to provide a device for manufacturing an absorbent article capable of arranging a band-shaped elastic member while adjusting its shape to a fine curved shape so as to achieve further improvement in the fitting feeling.

In order to solve the problems described above , according to the present disclosure, a device for manufacturing an absorbent article (a device 100 for manufacturing an absorbent article), configured to install a band-shaped, leg-hole elastic member to be continuously conveyed, on a web in which components configuring the absorbent article are conveyed in continuity, the device comprising: a guide mechanism (a guide mechanism 110) configured to convey while displacing the elastic member in a crossing direction perpendicular to a conveyance direction of the elastic member; a rotation mechanism (a rotation mechanism 120) configured to convey the elastic member supplied from the guide mechanism, along an outer circumference surface while setting a rotation mechanism axis (a rotation mechanism axis 121a) as a rotation center; a cutting mechanism (a cutting mechanism 130) configured to cut the elastic member conveyed along the outer circumference surface of the rotation mechanism; and an installing mechanism (an installing mechanism 140) configured to intermittently install on the web, the elastic member cut by the cutting mechanism, wherein: the guide mechanism has: a first roller (a first roller 111) configured to convey the elastic member along the outer circumference surface while setting a first guide axis (a first guide axis 111a) as a rotation center; a second roller (a second roller 112) arranged downstream in a conveyance direction of the first roller, and configured to convey the elastic member supplied from the first roller, along the outer circumference surface while setting a second guide axis (a second guide axis 112a) as a rotation center; and a swinging member (a swinging member 113) being an elongated member in a plate shape extending in the conveyance direction of the elastic member, having one end connected to a driving member and the other end connected to the second roller; the swinging member is configured to support the first roller and the second roller rotatably and to swing the second roller in the crossing direction while setting as a rotation center, a swinging axis (a swing axis 113a) in a supplying direction of the elastic member to the first roller; the rotation mechanism has: a plurality of holding members (a plurality of holding members 122) arranged at a predetermined interval in a circumferential direction on the outer circumference surface of the rotation mechanism, and configured to convey the elastic member from a first position receiving the elastic member to a second position passing the elastic member to the installing mechanism; and a rotation driving member configured to shift each of the holding members; the rotation driving member is configured to shift the holding members so that an interval between the holding members is made wider in the second position than that in the first position by changing a speed of the holding members; and the rotation mechanism includes a rotation member configured to rotate the holding members around an axis (axis 122e) which is perpendicular to the outer circumference surface and extends in a radial direction of the rotation mechanism; the guide mechanism is configured to convey the elastic member to the rotation mechanism while displacing the elastic member in the crossing direction such that the elastic member thus conveyed is arranged in a curved shape on the outer circumference surface of the rotation mechanism; the rotation mechanism is configured to convey the elastic member in the conveyance direction while rotating the elastic member along the outer circumference surface; the cutting mechanism is configured to cut the elastic member conveyed by the rotation mechanism with respect to each of the holding members; and the installing mechanism is configured to intermittently install on the web the elastic member cut by the cutting mechanism; wherein the elastic member is inclined with respect to the conveyance direction upon rotation of the holding members around the holding rotation axis so as to lead to a difference in a position in the crossing direction between a front end of the elastic member and a back end of the elastic member.

### [Brief Description of Drawings]

[Fig. 1] Fig. 1 is a plan view of an absorbent article according to a first embodiment.
[Fig. 2] Fig. 2 is a side view showing a device for manufacturing the absorbent article according to the first embodiment, the device including a rotation mechanism as a reference example.
[Fig. 3] Fig. 3 is a side view of a guide mechanism shown in Fig. 2.
[Fig. 4] Fig. 4 is a fragmentary view taken in the direction of arrow A-A of Fig. 3.
[Fig. 5] Fig. 5 is a view showing the rotation mechanism shown in Fig. 2. Fig. 5(a) is a side view and Fig. 5(b) is a fragmentary view taken in the direction of arrow B-B of Fig. 5(a).
[Fig. 6] Fig. 6 is a plan view of elastic members in a conveyance direction to illustrate a manufacturing step by a device for manufacturing an absorbent article shown in Fig. 2.
[Fig. 7] Fig. 7 (reference example) is a side view of the device for manufacturing an absorbent article according to the second embodiment.
[Fig. 8] Fig. 8 is a view showing a rotation mechanism of a device for manufacturing an absorbent article according to a third embodiment. Fig. 8(a) is a side view and Fig. 8(b) and Fig. 8(c) are fragmentary views taken in the direction of arrow C-C of Fig. 8(a).
[Fig. 9] Fig. 9 is a view for illustrating operation of a pin member.
[Fig. 10] Fig. 10 is a plan view of elastic members in a conveyance direction to illustrate a manufacturing step by the device for manufacturing the absorbent article according to the third embodiment.
[Fig. 11] Fig. 11 is a plan view of elastic members in a conveyance direction to illustrate a manufacturing step by a device for manufacturing an absorbent article according to a fourth embodiment.

### [Description of Embodiments]

Hereinafter, a device for manufacturing an absorbent article according to the present invention is explained with reference to drawings. Specifically, (1) Configuration of absorbent article, (2) Method of manufacturing absorbent article, (3) Configuration of device for manufacturing absorbent article, (4) Configuration of guide mechanism, (5) Configuration of rotation mechanism (reference example), (6) Step of forming elastic member by device for manufacturing absorbent article, (7) Configuration of device for manufacturing absorbent article according to second embodiment, (8) Configuration of device for manufacturing absorbent article according to third embodiment, (9) Method of manufacturing absorbent article according to fourth embodiment, and (10) Other embodiment are explained.

In the following description of the drawings, the same or similar reference numerals are used to designate the same or similar parts. It will be appreciated that the drawings are schematically shown and the ratio and the like of each dimension are different from the real ones. Therefore, a specific dimension should be determined in view of the following description. Moreover, among the drawings, the respective dimensional relations or ratios may naturally differ.

### (1) Configuration of absorbent article

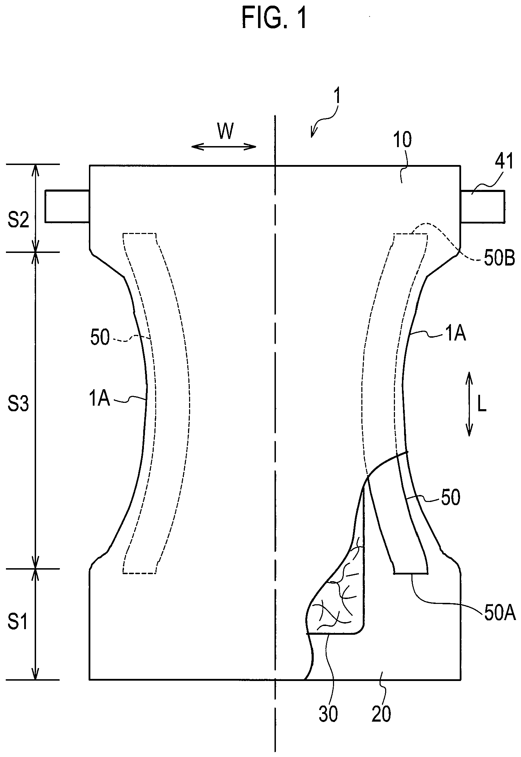

First of all, a configuration of an absorbent article 1 according to the present embodiment is explained with reference to drawings. Fig. 1 is a plan view showing the absorbent article 1 according to the present embodiment. In the present embodiment, the absorbent article 1 is an open-type disposable diaper.

In the present embodiment, the open- type diaper means a diaper that is in a spread-out state prior to use, rather than being formed in the shape of pants beforehand, and is worn by a wearer by fastening the predetermined portions of the product to each other with a tape and the like.

The absorbent article 1 has a front waistline region S1 corresponding to the front waistline of the wearer, a back waistline region S2 corresponding to the back waistline of the wearer, and a crotch region S3 corresponding to the crotch of the wearer and positioned between the front waistline region S1 and the back waistline region S2, in a longitudinal direction L of the disposable diaper 1. The disposable diaper 1 includes a topsheet 10, an exterior sheet 20, and the absorber 30.

The topsheet 10 is provided on the side that is in contact with the skin of the wearer. The topsheet 10 is formed by a liquid-permeable sheet, such as a hydrophilic nonwoven cloth and woven cloth, an aperture plastic film, or an aperture hydrophobic nonwoven cloth.

The exterior sheet 20 has a back nonwoven fabric that is in contact with the clothing, and a liquid-impermeable film (hereinafter, referred to as a back film) positioned towards the skin from the back nonwoven fabric and formed by a water-resistive film (for example, polyethylene). The back film is made from a moisture-permeable or moisture-impermeable film.

The absorber 30 is provided between the topsheet 10 and the exterior sheet 20. The absorber 30 absorbs bodily waste such as bodily fluid of the wearer. The absorber 30 is formed from an absorbent core such as ground pulp and high absorbent polymer, and an absorbent sheet such as a tissue for covering the absorbent core.

Both sides 1A defined as the ends in the widthwise direction of the topsheet 10 and the exterior sheet 20 cave in towards the inner side in the widthwise direction in the crotch region S3. The regions around the concaves of the crotch region S3 become leg-hole regions arranged along legs of the wearer. In the leg-hole regions, a pair of leg-hole elastic members 50 forming leg-hole gathers is formed.

The leg-hole elastic member 50 is formed in a band shape having a predetermined width and is arranged in an elongated state in a longitudinal direction L. The leg-hole elastic member 50 is arranged in a curved shape which curves further outward in a widthwise direction as it extends towards a front end 50A and a back end 50B respectively positioned on the outer sides in the longitudinal direction from the center in the longitudinal direction L.

A fixing member 41 configured to be fixed to the exterior sheet 20 in the front waistline region S1 is provided in the back waistline region S2. The fixing member 41 is joined with the exterior sheet 20. The fixing member 41 is formed by a hook-and-loop fastener and an adhesive tape.

### (2) Method of manufacturing absorbent article

Next, an example of a method of manufacturing the absorbent article according to the present embodiment is explained. As far as the method that is not described in the present embodiment is concerned, the existing method can be used. Furthermore, the manufacturing method explained below is only an example, and the absorbent article can also be manufactured by other manufacturing methods. The method of manufacturing the absorbent article includes at least a component-forming step, a component-loading step, a leg-hole-forming step, and a cutting step.

In the component-forming step, components configuring the absorbent article are formed. Specifically, for example, an absorbent material configuring the absorber is deposited to form the absorber 30, and after arranging a continuous band-shaped elastic member in a predetermined shape, the elastic member is cut into an individual product length to form the leg-hole elastic member 50.

In the component-loading step, on the web configuring the exterior sheet 20, components configuring the absorbent article 1 are loaded, in which the components include, e.g., the leg-hole elastic member 50, other web such as a web configuring the topsheet, a water-resistive sheet, and the absorber.

In the leg-hole-forming step, the topsheet 10 and the exterior sheet 20 are cut along the concaves configuring the leg-hole regions outside the leg-hole elastic members 50 in the widthwise direction. In this manner, the leg-hole regions arranged along the legs of the wearer are formed.

In the cutting step, a continuous body in which the topsheet 10, the exterior sheet 20, the absorber 30, and the leg-hole elastic members 50 are arranged is cut into a size of a single product in the widthwise direction W. In this manner, the absorbent article 1 is manufactured.

### (3) Configuration of device for manufacturing absorbent article

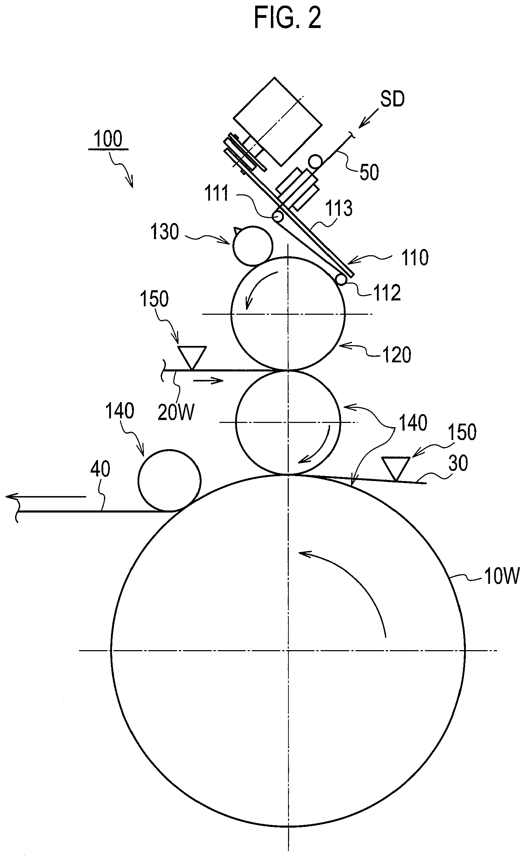

Next, a configuration of a device for manufacturing the absorbent article, used in the component-forming step and the component-loading step described above is explained in detail. Fig. 2 is a side view schematically showing a device 100 for manufacturing the absorbent article. The device 100 for manufacturing the absorbent article has a guide mechanism 110, a rotation mechanism 120, a cutting mechanism 130, an installing mechanism 140, and an adhesive-application mechanism 150. The device 100 for manufacturing the absorbent article is a device configured to install the band-shaped leg-hole elastic member 50 which is to be continuously conveyed, on the web in which the components configuring the absorbent article are conveyed in continuity.

The guide mechanism 110 feeds the leg-hole elastic member 50 towards the rotation mechanism 120 while swinging the leg-hole elastic member 50 in the crossing direction CD at a predetermined period. Accordingly, the leg-hole elastic member 50 is conveyed to the rotation mechanism 120 while being displaced in the crossing direction CD. The leg-hole elastic member 50 thus fed is arranged in a curved shape on the outer circumference surface of the rotation mechanism 120.

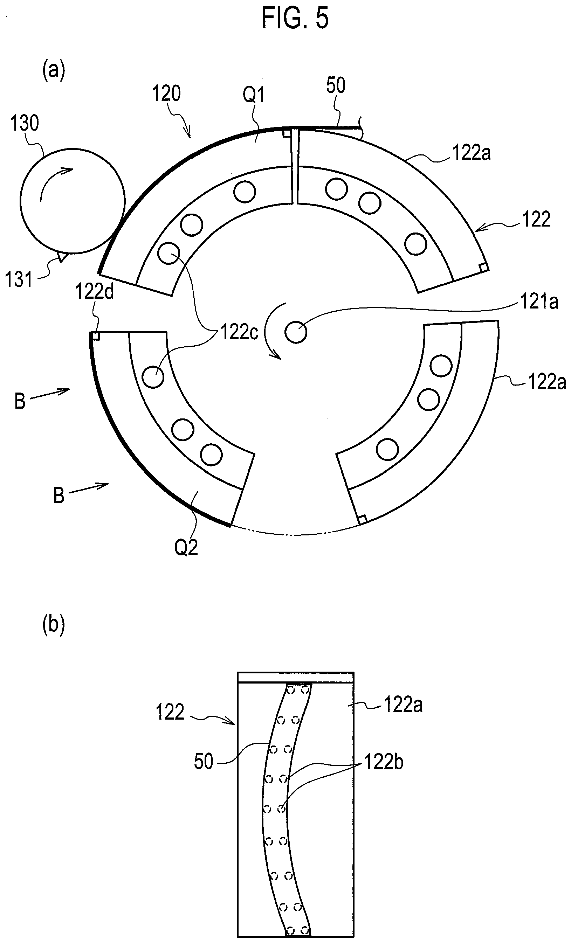

The rotation mechanism 120 feeds the leg-hole elastic member 50 towards the installing mechanism 140 while maintaining a shape of the leg-hole elastic member 50 supplied from the guide mechanism 110. The rotation mechanism 120 has a plurality of holding members 122 which rotate around a rotation mechanism axis 121a, and conveys the leg-hole elastic member 50 through the holding members 122 (see, Fig. 5).

The cutting mechanism 130 cuts the leg-hole elastic member 50 conveyed by the rotation mechanism 120, with respect to each holding member 122. The leg-hole elastic member 50 cut by the cutting mechanism 130 attains its installation length of an individual product.

The installing mechanism 140 includes a plurality of press rollers and joins respective components together by sandwiching the components between the rollers. Specifically, for example, the leg-hole elastic member 50 cut by the cutting mechanism 130 is installed intermittently on an exterior sheet web 20W in which the exterior sheets 20 are continuously conveyed.

The adhesive-application mechanism 150 is arranged upstream of the installing mechanism 140 and defined as a spray-type device configured to apply the adhesive (hot melt adhesive, for example) on a web such as the exterior sheet web 20W.

After adhesion of the leg-hole elastic member 50 to the exterior sheet, the installing mechanism 140 attaches the absorber 30 to which the adhesive-application mechanism 150 applies the adhesive, and a topsheet web 10W in which the topsheets 10 are in continuity. In this manner, a continuous body 40 is formed, in which the absorbent articles are in continuity in the longitudinal direction L.

In the present embodiment, the leg-hole elastic member 50 is installed on the exterior sheet web 20W. However, the leg-hole elastic member 50 is not limited to this configuration, and may be arranged on a web configuring a side sheet in a case of the absorbent article having the side sheet, or may be installed on other web.

### (4) Configuration of guide mechanism

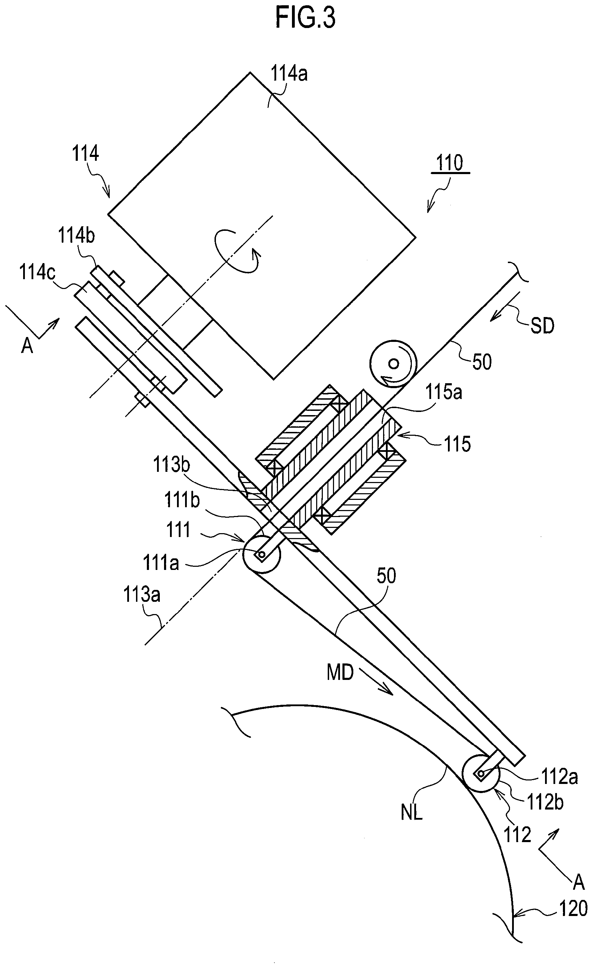

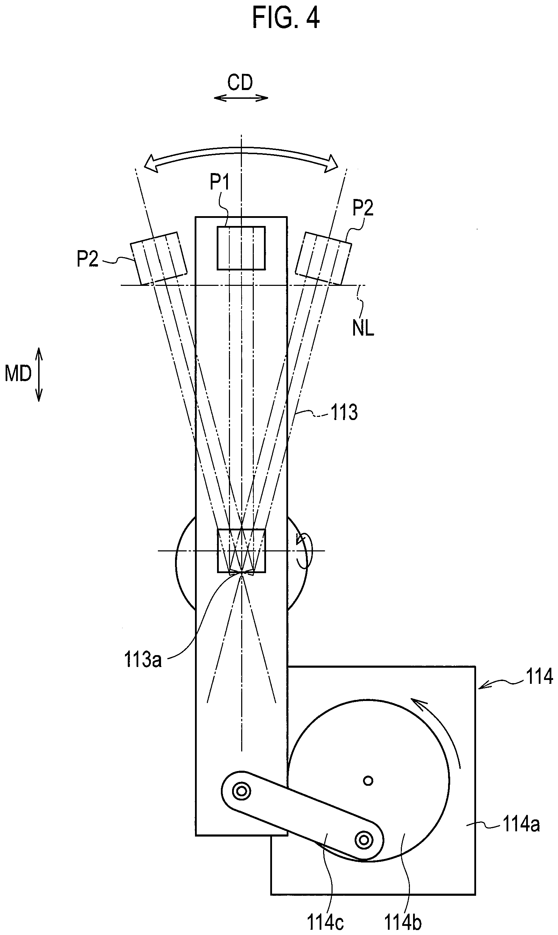

Next, the guide mechanism 110 is explained in detail based on Figs. 3 and 4. Fig. 3 is a side view of the guide mechanism 110 and Fig. 4 is a fragmentary view taken in the direction of arrow A-A of Fig. 3.

The guide mechanism 110 has: a first roller 111 which conveys the leg-hole elastic member 50 along an outer circumference surface 111b while setting a first guide axis 111a as a rotation center; a second roller 112 which is arranged downstream of the first roller 111 in a conveyance direction MD, and conveys the leg-hole elastic member 50 supplied from the first roller 111, along an outer circumference surface 112b while setting a second guide axis 112a as a rotation center; a swinging member 113 which supports the first roller 111 and the second roller 112 rotatably, and swings the second roller 112 in the crossing direction CD while setting as a rotation center a swinging axis 113a in a supplying direction SD of the elastic member to the first roller 111; and a driving member 114 which drives the swinging member 113.

The swinging member 113 is an elongated member in a plate shape extending in a conveyance direction of the leg-hole elastic member 50. The swinging member 113 has one end connected to the driving member 114 and the other end connected to the second roller 112. The driving member 114 has: a motor 114a configured to perform rotary drive; a crank disk 114b connected directly to a rotation axis of the motor 114a; and a connecting rod 114c configured to connect the crank disk 114b and the swinging member 113. The driving member 114 swings the swinging member 113 in the crossing direction CD in accordance with rotation of the motor 114a. Each of the ends of the swinging member 113 swings in the crossing direction CD while setting the rotation axis 113a as a rotation center.

One of the elongated surfaces of the swinging member 113 is arranged so as to face the rotation mechanism 120 and on this surface, the first roller 111 and the second roller 112 are supported rotatably. The other surface is arranged so as to face an elastic-member-supplying mechanism, not shown, and with this surface, a shaft member 115 configuring a rotation axis of the swinging member 113 is joined.

The shaft member 115 is a cylindrical body provided with a hollow part 115a, and is protruded from the swinging member 113 towards the elastic-member-supplying mechanism side. The swinging member 113 is provided with a through-hole 113b corresponding to the hollow part 115a of the shaft member 115. The band-shaped elastic member supplied from an elastic-member-supplying device, not shown, passes through the hollow part 115a of the shaft member 115 and passes through the through-hole 113b of the swinging member 113, thereby being guided to the first roller 111.

The elastic member supplied from the elastic-member-supplying device is guided to the second roller 112 along the outer circumference surface 111b of the first roller 111, thereby being guided to the rotation mechanism 120 from the second roller 112. As shown in Fig. 4, the swinging member 113 swings with the swinging axis 113a being set as the rotation center.

The second roller 112 reciprocates between a center position P1 as a swinging center and outer end positions P2 as both ends in the crossing direction. The leg-hole elastic member 50 is arranged in a wave shape within a predetermined range in the crossing direction CD. Reference NL shown in Fig. 4 designates a contact line which virtually shows a position of a contact point at which the leg-hole elastic member 50 conveyed from the second roller 112 comes in contact with the rotation mechanism 120.

In a state where the second roller 112 is positioned in the center position P1, the leg-hole elastic member 50 is arranged at the center in the crossing direction within the predetermined range. On the other hand, in a state where the second roller 112 is positioned in the outer end position P2, the leg-hole elastic member 50 is arranged at the outer end in the crossing direction within the predetermined range. The present embodiment is configured so that the contact line NL is arranged directly below the second roller 112 in a state where the second roller 112 is positioned in the outer end position P2.

For example, when the second roller 112 and the contact line NL are separated from each other in a state where the second roller 112 is positioned in the outer end position P2, the misalignment amount becomes large in the crossing direction between the second roller as an elastic-member feeding position and the contact point of the contact line. In a state where the second roller 112 is positioned in the outer end position P2, a rotation angle of the swinging member 113 is large and the misalignment amount becomes large in the crossing direction between the second roller as the elastic-member feeding position and the contact point. Therefore, in a state where the second roller 112 is positioned in the outer end position P2, it is desirable that the contact line NL be provided adjacent to the second roller 112.

When the contact line NL is positioned in a side (downside in Fig. 4) which is separated from the second roller 112 and closer to the swinging axis 113a, a distance in the crossing direction between the feeding position and the contact point becomes longer, thereby requiring the swinging member to have a larger swinging angle. Therefore, the misalignment amount between a position of the second roller for feeding the leg-hole elastic member and the contact point becomes larger, thereby leading to the possibility that a fine curved shape cannot be achieved.

However, in the present embodiment, the contact line NL is arranged between the second roller 112 and the swinging axis 113a, and also is arranged directly below the second roller 112 in the outer end position P2 so as to reduce the swinging angle of the swinging member at the ends in the crossing direction of the curved shape, thereby achieving a fine curved shape.

Further, since the swinging axis 113a of the swinging member 113 and the conveyance direction SD of the elastic member correspond to each other, the first roller to which the supplied elastic member is guided is positioned in the swinging center of the swinging member. Therefore, the displacement amount of the first roller following the swing of swinging member is so small that the elastic member hardly drops from the first roller. Thus, stable conveyance of the leg-hole elastic member can be achieved and a fine displacement of the leg-hole elastic member can be performed by the first and second rollers. This stable conveyance of the elastic member increases the adjustment accuracy of the elastic member, thereby realizing a fine curved shape.

### (5) Configuration of rotation mechanism (reference example)

Next, the rotation mechanism 120 is described in detail based on Fig. 5. Fig. 5(a) is a schematic side view of the rotation mechanism 120 and Fig. 5(b) is a fragmentary view taken in the direction of arrow B-B of Fig. 5(a). The rotation mechanism 120 of the first embodiment is a reference example in the sense that it does not include a rotation member configured to rotate the holding members around an axis which is perpendicular to the outer circumference surface and extends in a radial direction of the rotation mechanism.

The rotation mechanism 120 includes: the plurality of holding members 122 arranged at predetermined intervals in a circumferential direction on the outer circumference surface of the rotation mechanism; and a rotation driving member (not shown) configured to rotate the holding members 122. The rotation mechanism 120 can be configured from, for example, an elastic-band attaching device described in Japanese Patent No. 2609252.

The leg-hole elastic member 50 conveyed by the holding members 122 is cut into each predetermined length by a cutting blade 131 of the cutting mechanism 130 during a conveyance process. The holding member 122 is provided with a receiving blade 122d in a position facing the cutting blade 131. The cutting blade 131 and the receiving blade 122d are fitted to each other so as to cut the leg-hole elastic member 50.

The rotation driving member shifts each of the holding members 122 in the circumferential direction while setting the rotation mechanism axis 121a as the center. The holding members 122 convey the leg-hole elastic member 50 from a first position Q1 which receives the leg-hole elastic member 50 from the guide mechanism 110, to a second position Q2 which passes the leg-hole elastic member 50 to the installing mechanism 140.

The rotation driving member shifts the holding members 122 while changing a speed thereof. The rotation driving member shifts the holding member so that an interval between the holding members is wider in the second position than that in the first position. Specifically, a shifting speed of the holding member 122 is accelerated from the first position to the second position so as to extend a distance between the holding members. After the leg-hole elastic member 50 is transferred to the installing mechanism 140 in the second position, the shifting speed of the holding members 122 is decelerated to shorten the distance between the holding members. This adjustment of a distance between the holding members enables the leg-hole elastic members 50 to be arranged intermittently at predetermined intervals.

As shown in Fig. 5(b), the holding member 122 is provided with holes 122b in a contact surface 122a which comes in contact with the leg-hole elastic member 50. The rotation mechanism 120 includes a suction means, not shown, and suctions the leg-hole elastic member 50 on the contact surface 122a, through an air hole 122c formed in a side surface of the holding member 122 and the hole 122b formed in the contact surface 122a. This holding of the elastic member on the contact surface by the suction member enables the elastic member to be conveyed while retaining its shape, so that the elastic member can be conveyed to the installing mechanism while maintaining a fine curved shape.

### (6) Step of forming elastic member by device for manufacturing absorbent article

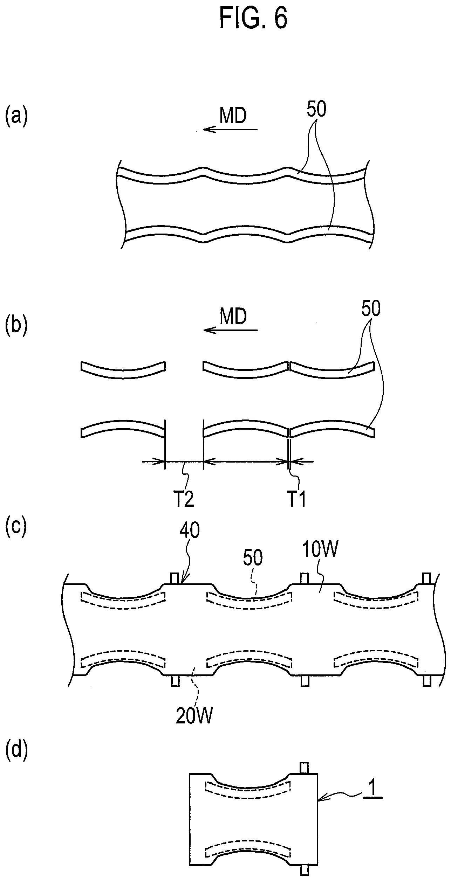

Next, a step of forming the elastic member by the device 100 for manufacturing the absorbent article described above is explained based on Fig. 6. Fig. 6 is a schematic plan view of the absorbent article and the components in the conveyance direction of the components of the absorbent article. The elastic member supplied from the elastic-member-supplying device is swung in the crossing direction by the guide member 110 as shown in Fig. 6(a) and is arranged in a curved shape on the holding members 122 of the rotation mechanism 120.

Next, the leg-hole elastic member 50 is cut by the cutting mechanism 130 during a conveyance process by the rotation mechanism 120, as shown in Fig. 6(b). An interval between the adjacent elastic members is extended in accordance with a speed change of the holding members 122 (T1 < T2).

The leg-hole member 50 is adhered in an elongated state between the exterior sheet web 20W and the topsheet web 10W by the installing mechanism 140. The leg-hole elastic member 50 is arranged intermittently in the conveyance direction MD. Subsequently, the exterior sheet web 20W and the topsheet web 10W are cut along the leg-hole regions, thereby forming the continuous body 40 of the absorbent article provided with the leg-hole regions shown in Fig. 6(c). The continuous body 40 of the absorbent article is cut in the crossing direction, thereby forming the absorbent article 1 shown in Fig. 6(d). The absorbent article 1 manufactured as described above has the band-shaped elastic members arranged intermittently in a fine curved shape, thereby achieving further improvement in the fitting feeling of the leg-hole regions.

### (7) Configuration of device for manufacturing absorbent article according to second embodiment (reference example)

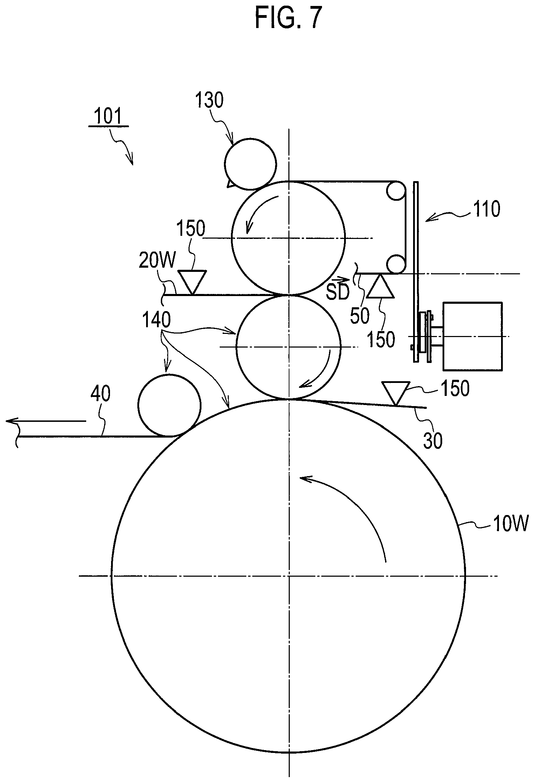

Next, a device 101 for manufacturing an absorbent article according to the second embodiment is explained with reference to Fig. 7. In the following explanation of the embodiment, only the configuration that is different from the first embodiment is explained, and the same signs are used for the configuration that is the same as the first embodiment while omitting the explanation.

In the device 101 for manufacturing the absorbent article according to the second embodiment, the adhesive-application mechanism 150 which applies the adhesive to the elastic member supplied from the elastic-member-supplying device is arranged upstream of the guide mechanism 110. By providing the adhesive-application mechanism 150 upstream of the guide mechanism 110, the adhesive can be applied to the entire elastic member, thereby making it easy for the leg-hole elastic member 50 to maintain its shape when transferring to the web.

It is desirable that the adhesive be applied to the entire elastic member 50, especially to parts positioned in the ends in a cutoff state. Precise arrangement of the ends of the leg-hole elastic member 50 makes it easier for the leg-hole elastic member 50 to maintain a fine curved shape.

Further, with the arrangement of the guide mechanism 110 according to the second embodiment, the adhesive can be applied to the leg-hole elastic member 50 before the leg-hole elastic member 50 is supplied to the guide mechanism 110, so that the leg-hole elastic member 50 can be arranged in a fine curved shape by being swung in the crossing direction CD in a state where the adhesive is applied to the elastic member 50.

### (8) Configuration of device for manufacturing absorbent article according to third embodiment

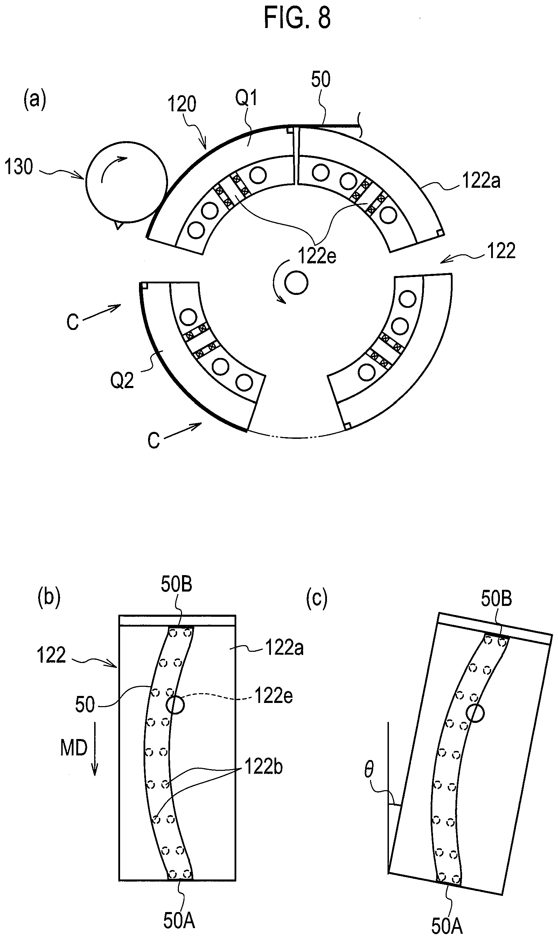

Next, a device for manufacturing an absorbent article according to the third embodiment is explained. Compared with the first embodiment, the manufacturing device according to the third embodiment has a different configuration in a rotation mechanism. Fig. 8 is a view showing a rotation mechanism according to the third embodiment. Fig. 8(a) is a side view and Figs 8(b) and 8(c) are views showing a rotation operation.

The rotation mechanism according to the third embodiment includes a rotation member (not shown) which rotates the holding members around a holding rotation axis 122e which crosses the outer circumference surface and extends in a radial direction of the rotation mechanism. The holding members are configured to be rotatable around the holding rotation axis 122e.

Fig. 8(b) is a view showing a rotation operation of the holding member 122 as viewed in the direction of arrow C-C of Fig. 8(a). Fig. 8(b) shows a state before rotation around the holding rotation axis 122e. Fig. 8(c) is a state after rotation at an angle of θ degrees around the holding rotation axis 122e. The leg-hole elastic member 50 is inclined with respect to the conveyance direction MD upon rotation of the holding members 122 around the holding rotation axis 122e.

The elastic member can be rotated by the rotation mechanism, thereby achieving a more complicated shape. Especially in a case of manufacturing the absorbent articles which are continuous in the longitudinal direction, if the leg-hole elastic member 50 is not rotated, the back end 50B of the leg-hole elastic member 50 becomes the front end 50A of the leg-hole elastic member 50 of the subsequent product, so that the front end 50A of the leg-hole elastic member 50 has the same position as that of the back end 50B in the crossing direction. However, rotation of the leg-hole elastic member 50 leads to a difference in a position in the crossing direction between the front end 50A of the leg-hole elastic member 50 and the back end 50B, thereby achieving a more complicated shape.

Further, the rotation mechanism according to the third embodiment includes: a pin member 123 which is protruded from the contact surface 122a through the hole 122b formed in the contact surface 122a of the holding member 122, and is inserted into the leg-hole elastic member 50 which is in contact with the contact surface 122a; and a driving member (not shown) by which the pin member 123 is protruded from the contact surface.

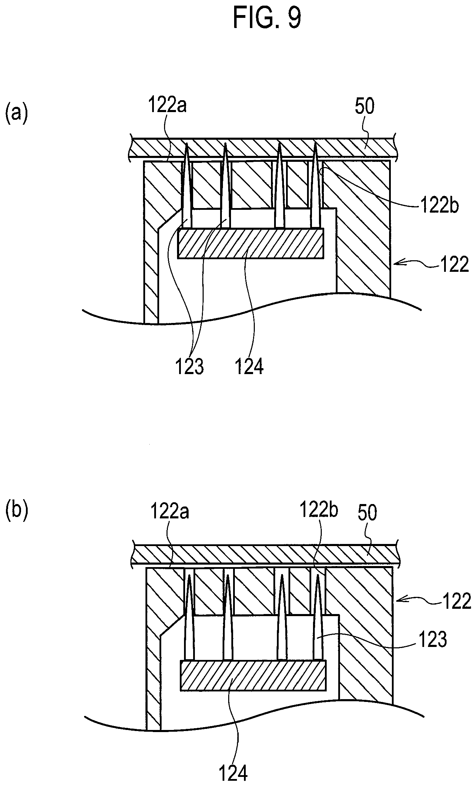

The pin member 123 is connected to a plate unit 124 in a plate shape and formed so as to protrude from this plate unit 124. The pin member 123 realizes a state where it is protruded from the contact surface 122a of the holding member 122 and inserted into the leg-hole elastic member 50, as shown in Fig. 9(a) and a state where the pin member 123 is arranged on the inside with respect to the contact surface 122a of the holding member 122 and released from the leg-hole elastic member 50.

The pin member is protruded from the contact surface when shifting from the first position to the second position, and in the second position, the pin member is driven so as to be arranged on the inside with respect to the contact surface. Therefore, the elastic member can be conveyed by the rotation mechanism in a state where a fine curved shape of the elastic member is maintained, by holding the elastic member by means of the pin member. Further, in the second position, the pin member is pulled out from the elastic member, so that the elastic member is passed to the web side smoothly.

In the present embodiment, the pin member is shifted to realize a state where the pin member is protruded from the contact surface and a state where the pin member is arranged on the inside with respect to the contact surface. However, the contact surface of the holding member may be shifted to realize a state where the pin member is protruded from the contact surface and a state where the pin member is arranged on the inside with respect to the contact surface.

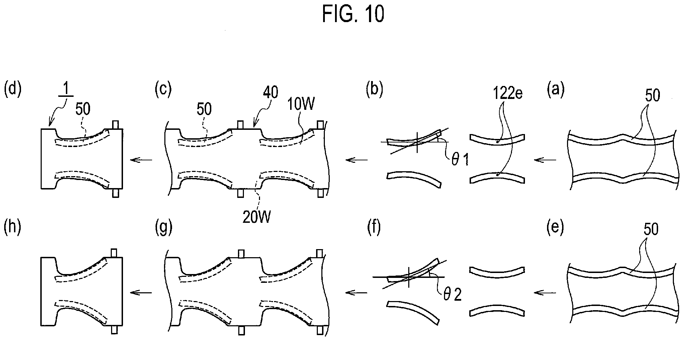

Fig. 10 is a view illustrating a step of forming the elastic member by the third device for manufacturing the absorbent article. In Figs. 10(a) to 10(d), the elastic member is rotated at an angle of θ1 degrees. In Figs. 10(e) to 10(h), the elastic member is rotated at an angle of θ2 degrees. This rotation of the elastic member leads to a difference in a position in the crossing direction between the front end 50A of the elastic member and the back end 50B, so that the leg-hole elastic member can be arranged in various patterns.

### (9) Method of manufacturing absorbent article according to fourth embodiment

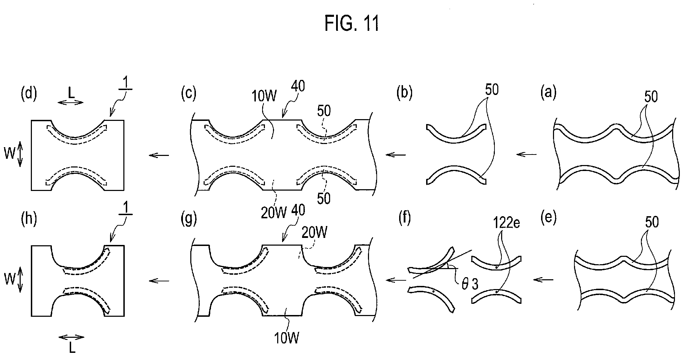

Fig. 11 is a view illustrating a step of forming an elastic member by a device for manufacturing an absorbent article according to the fourth embodiment. The absorbent article according to the fourth embodiment is a pant-type diaper and is manufactured in continuity in a widthwise direction. In Figs. 11(a) to 11(d), the elastic member is not rotated. In Figs. 11(e) to 11(h), the elastic member is rotated at an angle of θ3 degrees. Although it is not shown, subsequently to Figs. 11(d) and 11(h), the absorbent article is folded over in a longitudinal direction to join the sides 1A of the absorbent article, which are to be arranged around the waistline of the wearer, thereby manufacturing the pant-type absorbent article. As described above, not only in the open-type absorbent article but also in the pant-type absorbent article, the band-shaped elastic member can be arranged in a fine curved shape.

### (10) Other embodiment

So far, the present invention as defined by the appended claims is disclosed through the above embodiments. However, it should not be interpreted that the statements and drawings configuring a part of the present disclosure limit the present invention.

For example, in a case of manufacturing absorbent articles in different sizes, the absorbent articles in different sizes can be manufactured by changing the rotation mechanism. Specifically, for example, the curvature radius of the rotation mechanism is changed by changing the holding members so as to change a length in the conveyance direction, thereby being able to manufacture the absorbent articles in different sizes.

As described above, needless to say, the present invention includes various embodiments and the like not described here.

This application is based on Japanese Patent Application No. 2011-189114 filed with the Japan Patent Office on August 31, 2011.
Industrial Applicability

According to a device for manufacturing an absorbent article in the present disclosure, an elastic member is continuously conveyed while being displaced in a crossing direction by means of a guide mechanism, so that the elastic member can be arranged in a fine curved shape. Further, the guide mechanism supports first and second rollers rotatably and further, includes a swinging member configured to swing the second roller in the crossing direction. Therefore, when swinging the second roller, a conveyance direction of the first roller can also be changed following the swing, so that the elastic member can be arranged in a curved shape while preventing the band-shaped elastic member from dropping.

Further, the elastic member arranged in a curved shape by the guide mechanism is cut at a predetermined length by a cutting device during a conveyance step by a rotation mechanism. An interval between the elastic member cut into the predetermined length and the adjacent elastic member is extended during a conveyance process to a second position, and then the elastic member is installed on the web by an installing member. In this manner, the elastic members in a curved shape can be installed intermittently with a predetermined interval therebetween on the web. Thus, the band-shaped elastic members can be arranged in only the necessary portions while being adjusted to a fine curved shape and therefore, the absorbent article capable of achieving further improvement in the fitting feeling can be manufactured.
Reference Signs List

1:Absorbent article, 1A:Sides, 10:Topsheet, 10W:Topsheet web, 20:Exterior sheet, 20W:Exterior sheet web, 30:Absorber, 40:Continuous body, 50:Leg-hole elastic member, 100:Device for manufacturing absorbent article, 110:Guide mechanism, 111:First roller, 111a:First guide axis, 111b:Outer circumference surface, 112:Second roller, 112a:Second guide axis, 112b:Outer circumference surface, 113:Swinging member, 113a:Swinging axis, 113b:Through-hole, 114:Driving member, 115:Shaft member, 115a:Hollow part, 120:Rotation mechanism, 121a:Rotation mechanism axis, 122:Holding member, 122a:Contact surface, 122b:Holes, 122c:Air hole, 122d:Receiving blade, 123:Pin member, 130:Cutting mechanism, 131:Cutting blade, 140:Installing mechanism, 150:Adhesive-application mechanism, CD:Crossing direction, L:Longitudinal direction, MD:Conveyance direction, NL:Contact line, S1: Front waistline region, S2:Back waistline region, S3: Crotch region, SD:Conveyance direction of elastic member, W:Widthwise direction

## Claims

1. A device (100) for manufacturing an absorbent article, configured to install a band-shaped, leg-hole elastic member (50) to be continuously conveyed, on a web in which components configuring the absorbent article are conveyed in continuity, the device comprising:
a guide mechanism (110) configured to convey while displacing the elastic member in a crossing direction perpendicular to a conveyance direction of the elastic member;
a rotation mechanism (120) configured to convey the elastic member supplied from the guide mechanism, along an outer circumference surface while setting a rotation mechanism axis (121a) as a rotation center;
a cutting mechanism (130) configured to cut the elastic member conveyed along the outer circumference surface of the rotation mechanism; and
an installing mechanism (140) configured to intermittently install on the web the elastic member cut by the cutting mechanism, wherein:
the guide mechanism has:
a first roller (111) configured to convey the elastic member along the outer circumference surface while setting a first guide axis (111a) as a rotation center;
a second roller (112) arranged downstream in a conveyance direction of the first roller, and configured to convey the elastic member supplied from the first roller, along the outer circumference surface while setting a second guide axis (112a) as a rotation center; and
a swinging member (113) being an elongated member in a plate shape extending in the conveyance direction of the elastic member, having one end connected to a driving member (114) and the other end connected to the second roller (112);
the swinging member is configured to support the first roller and the second roller rotatably and to swing the second roller in the crossing direction, while setting as a rotation center a swinging axis (113a) in a supplying direction of the elastic member to the first roller;
the rotation mechanism (120) has:
a plurality of holding members (122) arranged at a predetermined interval in a circumferential direction on the outer circumference surface of the rotation mechanism, and configured to convey the elastic member from a first position (Q1) receiving the elastic member to a second position (Q2) passing the elastic member to the installing mechanism; and
a rotation driving member configured to shift each of the holding members;
the rotation driving member is configured to shift the holding members so that an interval between the holding members is made wider in the second position than that in the first position by changing a speed of the holding members; and
the rotation mechanism includes a rotation member configured to rotate the holding members around an axis (122e) which is perpendicular to the outer circumference surface and extends in a radial direction of the rotation mechanism;
the guide mechanism (110) is configured to convey the elastic member to the rotation mechanism while displacing the elastic member in the crossing direction such that the elastic member thus conveyed is arranged in a curved shape on the outer circumference surface of the rotation mechanism;
the rotation mechanism (120) is configured to convey the elastic member in the conveyance direction while rotating the elastic member along the outer circumference surface;
the cutting mechanism (130) is configured to cut the elastic member conveyed by the rotation mechanism with respect to each of the holding members; and
the installing mechanism (140) is configured to intermittently install on the web the elastic member cut by the cutting mechanism;
wherein the elastic member is inclined with respect to the conveyance direction upon rotation of the holding members (122) around the holding rotation axis (122e) so as to lead to a difference in a position in the crossing direction between a front end of the elastic member and a back end of the elastic member.

2. The device for manufacturing an absorbent article according to claim 1, wherein:
the rotation mechanism includes:
a pin member (123) configured to be protruded from a contact surface (122a) through a hole (122b) formed in the contact surface of the holding member and to be inserted into the elastic member (50) which is in contact with the contact surface; and
a relatively shifting member configured to relatively shift at least one of the contact surface of the holding member and the pin member with respect to the other; and
the relatively shifting member shifts the pin member in an axial direction of the hole of the contact surface by shifting at least one of the contact surface and the pin member with respect to the other, so as to realize a state in which the pin member is protruded from the contact surface and a state in which the pin member is arranged on an inside with respect to the contact surface.

3. The device for manufacturing an absorbent article according to claim 1, wherein:
a hole (122b) is formed in a contact surface (122a) of the holding member, which comes in contact with the elastic member (50); and
the rotation mechanism includes a suction member configured to suction the elastic member on the contact surface through the hole.

4. The device for manufacturing an absorbent article according to any one of claims 1 to 3, further comprising an adhesive application mechanism (150) configured to apply an adhesive to a contact surface of the elastic member conveyed by the guide mechanism, the contact surface coming in contact with the web.

## Patentansprüche

1. Vorrichtung (100) für die Herstellung eines absorbierenden Artikels, die zur Anbringung eines streifenförmigen, elastischen Beinöffnungselements (50), das kontinuierlich zu fördern ist, auf einer Bahn konfiguriert ist, in der die Komponenten, die den absorbierenden Artikel konfigurieren, kontinuierlich gefördert werden, wobei die Vorrichtung Folgendes umfasst:
einen Führungsmechanismus (110), der zur Förderung konfiguriert ist, während das elastische Element in einer Querrichtung senkrecht zu einer Förderrichtung des elastischen Elements verschoben wird;
einen Drehmechanismus (120), der zur Förderung des elastischen Elements, das von dem Führungsmechanismus geliefert wird, entlang einer äußeren Umfangsfläche konfiguriert ist, während eine Drehmechanismusachse (121a) als Drehzentrum festgelegt ist;
einen Schneidemechanismus (130), der zum Schneiden des elastischen Elements konfiguriert ist, das entlang der äußeren Umfangsfläche des Drehmechanismus gefördert wird; und
einen Anbringungsmechanismus (140), der zur unterbrochenen Anbringung des elastischen Elements, das durch den Schneidemechanismus geschnitten wurde, auf der Bahn konfiguriert ist, wobei:
der Führungsmechanismus Folgendes aufweist:
eine erste Rolle (111), die zur Förderung des elastischen Elements entlang der äußeren Umfangsfläche konfiguriert ist, während eine erste Führungsachse (111a) als Drehzentrum festgelegt ist;
eine zweite Rolle (112), die nachgelagert in einer Förderrichtung der ersten Rolle angeordnet ist und zur Förderung des elastischen Elements, das von der ersten Rolle geliefert wird, entlang einer äußeren Umfangsfläche konfiguriert ist, während eine zweite Führungsachse (112a) als Drehzentrum festgelegt ist; und
ein Schwenkelement (113), das ein längliches Element in einer Plattenform ist, das sich in der Förderrichtung des elastischen Elements erstreckt, ein Ende, das mit einem Antriebselement (114) verbunden ist, und das andere Ende, das mit der zweiten Rolle (112) verbunden ist, aufweist;
das Schwenkelement zur drehbaren Unterstützung der ersten Rolle und der zweiten Rolle und zum Schwenken der zweiten Rolle in der Querrichtung konfiguriert ist, während eine Schwenkachse (113a) in einer Laufrichtung des elastischen Elements zur ersten Rolle als Drehzentrum festgelegt ist;
der Drehmechanismus (120) Folgendes aufweist:
eine Vielzahl von Halteelementen (122), die in einem vorgegebenen Abstand in einer Umfangsrichtung auf der äußeren Umfangsfläche des Drehmechanismus angeordnet sind und zur Förderung des elastischen Elements von einer ersten Position (Q1), die das elastische Element empfängt, zu einer zweiten Position (Q2), die das elastische Element zum Anbringungsmechanismus weitergibt, konfiguriert sind; und
ein Drehantriebselement, das zur Verschiebung von jedem der Halteelemente konfiguriert ist;
das Drehantriebselement zur Verschiebung der Halteelemente derart konfiguriert ist, dass ein Abstand zwischen den Halteelementen in der zweiten Position breiter eingestellt wird als der in der ersten Position durch Änderung einer Geschwindigkeit der Halteelemente; und
der Drehmechanismus ein Drehelement einschließt, das zur Drehung der Halteelemente um eine Achse (122e) konfiguriert ist, die sich senkrecht zur äußeren Umfangsfläche befindet und in einer radialen Richtung des Drehmechanismus erstreckt;
der Führungsmechanismus (110) zur Förderung des elastischen Elements zum Drehmechanismus konfiguriert ist, während das elastische Element in einer Querrichtung derart verschoben wird, dass das so geförderte elastische Element in einer gebogenen Form auf der äußeren Umfangsfläche des Drehmechanismus angeordnet wird;
der Drehmechanismus (120) zur Förderung des elastischen Elements in der Förderrichtung konfiguriert ist, während das elastische Element entlang der äußeren Umfangsfläche gedreht wird;
der Schneidemechanismus (130) zum Schneiden des elastischen Elements konfiguriert ist, das durch den Drehmechanismus in Bezug auf jedes der Halteelemente gefördert wird; und
der Anbringungsmechanismus (140) zur unterbrochenen Anbringung des elastischen Elements, das durch den Schneidemechanismus geschnitten wurde, auf der Bahn konfiguriert ist;
wobei das elastische Element in Bezug auf die Förderrichtung bei Drehung der Halteelemente (122) um die Haltedrehachse (122e) geneigt ist, um zu einem Unterschied in der Position in der Querrichtung zwischen einem vorderen Ende des elastischen Elements und einem hinteren Ende des elastischen Elements zu führen.

2. Vorrichtung für die Herstellung eines absorbierenden Artikels nach Anspruch 1, wobei:
der Drehmechanismus Folgendes einschließt:
ein Stiftelement (123), das konfiguriert ist, um von einer Kontaktfläche (122a) durch ein Loch (122b), das in der Kontaktfläche des Halteelements ausgebildet ist, herausgestreckt zu werden und in das elastische Element (50), das mit der Kontaktfläche im Kontakt ist, eingeführt zu werden; und
ein relativ verschiebendes Element, das konfiguriert ist, um mindestens eine/s von der Kontaktfläche des Halteelements und dem Stiftelement in Bezug aufeinander zu verschieben; und
das relativ verschiebende Element das Stiftelement in einer axialen Richtung des Lochs der Kontaktfläche durch Verschiebung von mindestens einer/einem von der Kontaktfläche und dem Stiftelement in Bezug aufeinander verschiebt, um einen Zustand, indem das Stiftelement sich aus der Kontaktfläche herausstreckt, und einen Zustand, in dem das Stiftelement an einer Innenseite in Bezug auf die Kontaktfläche angeordnet ist, zu erzielen.

3. Vorrichtung für die Herstellung eines absorbierenden Artikels nach Anspruch 1, wobei:
ein Loch (122b) in einer Kontaktfläche (122a) des Halteelements ausgebildet ist, die mit dem elastischen Element (50) in Kontakt kommt; und
der Drehmechanismus ein Saugelement einschließt, das zum Ansaugen des elastischen Elements auf der Kontaktfläche durch das Loch konfiguriert ist.

4. Vorrichtung für die Herstellung eines absorbierenden Artikels nach einem der Ansprüche 1 bis 3, die weiter einen Haftmittelauftragungsmechanismus (150) umfasst, der zur Auftragung eines Haftmittels auf einer Kontaktfläche des elastischen Elements, das durch den Führungsmechanismus gefördert wird, konfiguriert ist, wobei die Kontaktfläche mit der Bahn in Kontakt kommt.

## Revendications

1. Dispositif (100) de fabrication d'un article absorbant, configuré à des fins d'installation d'un élément élastique de trou pour jambe en forme de bande (50) destiné à être transporté de manière continue, sur une bande de tissu dans laquelle des composants configurant l'article absorbant sont transportés en continu, le dispositif comportant :
un mécanisme de guidage (110) configuré pour transporter tout en le déplaçant l'élément élastique dans une direction allant dans le sens transversal perpendiculaire par rapport à une direction allant dans le sens du transport de l'élément élastique ;
un mécanisme de rotation (120) configuré pour transporter l'élément élastique alimenté en provenance du mécanisme de guidage, le long d'une surface circonférentielle extérieure tout en réglant un axe (121a) du mécanisme de rotation comme centre de rotation ;
un mécanisme de découpe (130) configuré pour découper l'élément élastique transporté le long de la surface circonférentielle extérieure du mécanisme de rotation ; et
un mécanisme d'installation (140) configuré pour installer de manière intermittente sur la bande de tissu l'élément élastique découpé par le mécanisme de découpe, dans lequel :
le mécanisme de guidage a :
un premier rouleau (111) configuré pour transporter l'élément élastique le long de la surface circonférentielle extérieure tout en réglant un premier axe de guidage (111a) comme centre de rotation ;
un deuxième rouleau (112) agencé en aval dans une direction allant dans le sens du transport du premier rouleau, et configuré pour transporter l'élément élastique alimenté en provenance du premier rouleau, le long de la surface circonférentielle extérieure tout en réglant un deuxième axe de guidage (112a) comme centre de rotation ; et
un élément de pivotement (113) qui est un élément allongé en une forme de plaque s'étendant dans la direction allant dans le sens du transport de l'élément élastique, ayant une extrémité raccordée à un élément d'entraînement (114) et l'autre extrémité raccordée au deuxième rouleau (112) ;
l'élément de pivotement est configuré pour supporter le premier rouleau et le deuxième rouleau de manière rotative et pour faire pivoter le deuxième rouleau dans une direction allant dans le sens transversal, tout en réglant comme centre de rotation un axe de pivotement (113a) dans une direction allant dans le sens de l'alimentation de l'élément élastique vers le premier rouleau ;
le mécanisme de rotation (120) a :
une pluralité d'éléments de retenue (122) agencés selon un intervalle prédéterminé dans une direction allant dans le sens de la circonférence sur la surface circonférentielle extérieure du mécanisme de rotation, et configurés pour transporter l'élément élastique depuis une première position (Q1) recevant l'élément élastique jusque sur une deuxième position (Q2) passant l'élément élastique jusqu'au mécanisme d'installation ; et
un élément d'entraînement de rotation configuré pour décaler chacun des éléments de retenue ;
l'élément d'entraînement de rotation est configuré pour décaler les éléments de retenue de telle sorte qu'un intervalle entre les éléments de retenue est rendu plus large dans la deuxième position par rapport à la première position par le changement d'une vitesse des éléments de retenue ; et
le mécanisme de rotation comprend un élément de rotation configuré pour faire tourner les éléments de retenue autour d'un axe (122e) qui est perpendiculaire par rapport à la surface circonférentielle extérieure et qui s'étend dans une direction allant dans le sens radial du mécanisme de rotation ;
le mécanisme de guidage (110) est configuré pour transporter l'élément élastique jusqu'au mécanisme de rotation tout en déplaçant l'élément élastique dans la direction allant dans le sens transversal de telle sorte que l'élément élastique ainsi transporté est agencé en une forme courbe sur la surface circonférentielle extérieure du mécanisme de rotation ;
le mécanisme de rotation (120) est configuré pour transporter l'élément élastique dans la direction allant dans le sens du transport tout en faisant tourner l'élément élastique le long de la surface circonférentielle extérieure ;
le mécanisme de découpe (130) est configuré pour découper l'élément élastique transporté par le mécanisme de rotation par rapport à chacun des éléments de retenue ; et
le mécanisme d'installation (140) est configuré pour installer de manière intermittente sur la bande de tissu l'élément élastique découpé par le mécanisme de découpe ;
dans lequel l'élément élastique est incliné par rapport à la direction allant dans le sens du transport lors de la rotation des éléments de retenue (122) autour de l'axe de rotation de retenue (122e) de manière à mener à une différence dans une position dans la direction allant dans le sens transversal entre une extrémité avant de l'élément élastique et une extrémité arrière de l'élément élastique.

2. Dispositif de fabrication d'un article absorbant selon la revendication 1, dans lequel :
le mécanisme de rotation comprend :
un élément formant broche (123) configuré pour faire saillie depuis une surface de contact (122a) au travers d'un trou (122b) formé dans la surface de contant de l'élément de retenue et pour être inséré dans l'élément élastique (50) qui est en contact avec la surface de contact ; et
un élément à décalage relatif configuré à des fins de décalage relatif d'au moins l'un parmi la surface de contact de l'élément de retenue et l'élément formant broche par rapport à l'autre ; et
l'élément à décalage relatif décale l'élément formant broche dans une direction allant dans le sens axial du trou de la surface de contact en décalant au moins l'un parmi la surface de contact et l'élément formant broche par rapport à l'autre, de manière à réaliser un état dans lequel l'élément formant broche fait saillie depuis la surface de contact et un état dans lequel l'élément formant broche est agencé sur une partie intérieure par rapport à la surface de contact.

3. Dispositif de fabrication d'un article absorbant selon la revendication 1, dans lequel :
un trou (122b) est formé dans une surface de contact (122a) de l'élément de retenue, qui entre en contact avec l'élément élastique (50) ; et
le mécanisme de rotation comprend un élément d'aspiration configuré pour aspirer l'élément élastique sur la surface de contact au travers du trou.

4. Dispositif de fabrication d'un article absorbant selon l'une quelconque des revendications 1 à 3, comportant par ailleurs un mécanisme d'application d'adhésif (150) configuré pour appliquer un adhésif sur une surface de contact de l'élément élastique transporté par le mécanisme de guidage, la surface de contact entrant en contact avec la bande de tissu.
